# EUROPÄISCHE PATENTSCHRIFT

(11) **EP 1 639 990 B1**
(45) Veröffentlichungstag und Bekanntmachung des Hinweises auf die Patenterteilung: **08.08.2012**
(21) Anmeldenummer: 05019947.0
(22) Anmeldetag: 14.09.2005
(51) Int. Cl.: A61K 8/73, A61K 8/97, A61Q 1/02, A61Q 3/02, A61Q 1/06

(54) **Reisstärke in kosmetischen Zusammensetzungen**
Rice starch in cosmetic compositions
Amidon de riz dans des formulations cosmétiques

(30) Priorität: 23.09.2004 DE 102004046125
(43) Veröffentlichungstag der Anmeldung: 29.03.2006
(73) Patentinhaber: Südzucker Aktiengesellschaft Mannheim/Ochsenfurt, 68165 Mannheim (DE)
(72) Erfinder: Braunagel, Alfred, 55128 Mainz (DE); Guderjahn, Lutz, Dr., 67591 Offstein (DE); Kowalczyk, Jörg, Dr., 67304 Eisenberg/Steinborn (DE); Walter, Thomas, Dr., 69242 Mühlhausen (DE)
(74) Vertreter: Schrell, Andreas

(56) Entgegenhaltungen:
- EP-A- 1 018 334
- EP-A- 1 064 908
- EP-A- 1 195 155
- WO-A-01/17488
- GB-A- 1 295 901
- US-B1- 6 383 475

## Beschreibung

Die vorliegende Erfindung betrifft Mittel und Verfahren zur Verbesserung des Farbauftrags von kosmetischen Zusammensetzungen. Die Erfindung betrifft auch kosmetische Zusammensetzungen, die bei ihrer Anwendung einen verbesserten Farbauftrag aufweisen.

Produkte der dekorativen Kosmetik werden weltweit verwendet. Dabei liegt es im Interesse des Anwenders, ein Kosmetikprodukt zu erhalten, welches unter verschiedenen Einsatzbedingungen leicht anzuwenden ist, und die Anwendung stets ein gleich bleibendes Ergebnis mit hoher Anmutung erzielt. Weiter will die Kosmetikindustrie dem Anwender von Kosmetikprodukten stets eine große Palette sowohl traditioneller als auch neuartiger dekorativer Effekte anbieten, die sich bezüglich ihrer Farbgebung, Farbschattierung, ihrem Glanz- und Lichteffekt und in weiteren Eigenschaften, die die Anmutung des aufgetragenen dekorativen Kosmetikums mitbestimmen, unterscheiden. Dabei liegt es im Interesse, Produkte bereitzustellen, die bei einfacher und unkomplizierter Anwendung optimale Ergebnisse erzielen.

Weiter müssen Produkte der dekorativen Kosmetik, das heißt Schminkprodukte, auch für den Auftrag auf empfindlichen Hautoberflächen wie den Lippen, den Augenlidern oder den Lidrändern geeignet sein. Dazu müssen die Produkte dieser Art beim Auftragen genügend weich sein. Gleichzeitig soll aber ein gleichmäßiger, kontrollierbarer, das heißt nicht zu starker oder zu schwacher, Farbauftrag erfolgen. Nach Möglichkeit soll das Kosmetikprodukt in einer leicht handhabbaren Form vorliegen und möglichst ohne zusätzliche Auftragevorrichtungen wie Pinsel, Schwämmchen, Bürste und ähnlichen Hilfsmitteln aufzutragen sein. Weiter sollen die Kosmetikprodukte genügend kompakt sein, wenn sie beispielsweise in Stiftform gegossen oder verpresst werden. Dabei soll gleichzeitig zur geforderten Weichheit und leichten Auftragbarkeit des Produkts eine große mechanische Widerstandsfähigkeit bei der Herstellung, Lagerung und beim Transport des Produkts gewährleistet sein, das heißt ein Absplittern, Zerbröseln oder Verlaufen des Produkts soll auch unter extremeren klimatischen Bedingungen oder mechanischen Belastungen nicht auftreten. Das aufgetragene Produkt soll außerdem gute Trageeigenschaften besitzen und weder zum Austrocknen neigen und Abblättern, noch die darunter liegende Haut oder Schleimhaut austrocknen oder reizen, noch den Flüssigkeitsaustausch der Haut oder Schleimhaut soweit beeinträchtigen, dass das aufgetragene Produkt verläuft oder in seiner Erscheinung beeinträchtigt wird.

Weitere Vorgaben an Produkte der dekorativen Kosmetik sind deren unbedingte medizinische Verträglichkeit sowie der gesteigerte Einsatz von nachwachsenden Rohstoffen als Bestandteile dieser Produkte, wobei insbesondere wünschenswert ist, dass diese Rohstoffe "in naturreiner Form" unmittelbar und ohne weitere Aufreinigungs- oder Syntheseschritte in den Produkten eingesetzt werden können. Eine entsprechende Auslobung bei der Vermarktung der Produkte wirkt dabei verkaufsfördernd.

Beispielsweise werden nachwachsende Rohstoffe als Bestandteile in Kosmetikprodukten und Körperpflegemitteln aus der Reispflanze (Oryza sativa) gewonnen. Dazu gehören Reiskeimmehl, Reiskleie, Reisstärken, Reisproteine, wachsartige Reisstärken sowie Öle und Fettsäuren aus Reiskeimen oder Reiskleie. Seit langem ist bekannt, Reisstärke als Grundlage für Körperpuder und Pudern der dekorativen Kosmetik sowie als Füllstoff oder Konsistenzgeber in Rouge, kosmetischen Gels, Cremes, kosmetischen Lösungen, kosmetischen Sprays sowie in Körperölen, Badeölen, -tabletten und -salzen sowie in Haarpflegeprodukten einzusetzen. Die Funktion der Reisstärke beschränkt sich dabei auf ihre Eigenschaft als Konsistenzgeber oder als Träger für Farb-, Duft- oder Wirkstoffe.

Das Dokument GB-A-1295901 offenbart die Verwendung pulverförmiger Trägermateralien unter anderem auch von lipophilisiertem Reis in dekorativer Kosmetik. *Die Lipophilisierung der Oberfläche des pulverförmigen Trägers wird durch chemische* Modifikation dieses Trägers erzielt.

Der färbende Anteil in Zusammensetzungen dekorativer Kosmetikprodukte besteht in der Regel in erster Linie aus mineralischen oder organischen Pigmenten sowie aus auch wasserlöslichen Farbstoffen, die beispielsweise auf puderartige Grundstoffe wie Kaolin, Aluminiumhydroxid oder Titandioxid niedergeschlagen werden. Zur Erzielung optimaler Anwendungseigenschaften, hohem Farbauftrag und hoher Deckkraft wird eine gleichmäßige feine Dispersion des Farbstoffanteils im Produkt angestrebt. Um eine hohe Dispersion zu erreichen, werden in der Regel heterozyklische Verbindungen und/oder fein dispergierte Metalloxide wie Eisenoxide eingesetzt.

Insbesondere in Verbindung mit Lippenstiften, deren Inhaltsstoffe in die Mundhöhle und damit auf Schleimhäute gelangen können, sowie mit Kosmetikprodukten, die auf den Lidrand aufgetragen werden, besteht die Gefahr, dass die Inhaltsstoffe der Zusammensetzungen in unerwünscht hohem Maße in den Körper aufgenommen werden und dort zu nachteiligen gesundheitlichen Effekten führen. So sind beispielsweise die in bekannten Kosmetikprodukten enthaltenen Metalloxide wie Eisenoxide oft mit anderen Metallsalzen und Metalloxiden verunreinigt, beispielsweise mit Nickelsalzen, die Allergie auslösend wirken Auch die weitere in bekannten Produkten zur Erhöhung der Dispersion der Farben beigefügten Komponenten wie heterozyklische Verbindungen weisen ein Allergie auslösendes Potential auf. Es ist daher auch wünschenswert, den Anteil von Komponenten, die gesundheitliche Risiken bergen, in Kosmetikprodukten zu reduzieren.

Das der vorliegenden Erfindung zugrunde liegende technische Problem besteht im Wesentlichen darin, verbesserte kosmetische Zusammensetzungen der dekorativen Kosmetik sowie Verfahren und Mittel zu deren Herstellung bereitzustellen, wobei die kosmetischen Zusammensetzungen die vorstehend skizzierten Nachteile des Standes der Technik überwinden und insbesondere verbesserte Eigenschaften bei ihrer Anwendung und Handhabung aufweisen.

Das technische Problem wird gemäß der Erfindung gelöst durch die Verwendung von Reisstärke als Bestandteil einer kosmetischen Zusammensetzung der dekorativen Kosmetik, welche insbesondere auf Haut, Haare oder Nägel aufgetragen wird, zum Zwecke der Verbesserung des Farbauftrags und insbesondere zur Steigerung der Deckkraft der aufgetragenen Zusammensetzung.

Die Erfinder fanden überraschend, dass der Zusatz von Reisstärke in färbenden, insbesondere farbpigmenthaltigen, kosmetischen Zusammensetzungen der dekorativen Kosmetik, beispielsweise in Lippenstift, den Farbauftrag unter normierten Applikationsbedingungen entscheidend verbessert. Dabei wird Reisstärke erfindungsgemäß als einzige Puderkomponente in der kosmetischen Zusammensetzung verwendet. Erfindungsgemäß wird Reisstärke in unbehandelter Form als native Reisstärke verwendet. Besonders vorteilhaft wird insbesondere die Gleichmäßigkeit des Auftrags der kosmetischen Zusammensetzung beziehungsweise des Farbauftrags verbessert. Besonders vorteilhaft wird insbesondere die Deckkraft des Farbauftrags verbessert. Besonders vorteilhaft wird weiter insbesondere die Menge an der Oberfläche von Haut, Haare oder Nägel aufgetragenem kosmetischer Zusammensetzung pro Anwendung erhöht. Besonders vorteilhaft wird schließlich insbesondere die Haftung der auf der Oberfläche von Haut, Haare oder Nägel aufgetragenen kosmetischen Zusammensetzung verbessert.

Im Zusammenhang mit der vorliegenden Erfindung wird unter einer Puderkomponente eine puderförmige Festphase bzw. pulvrige Komponente in der kosmetischen Zusammensetzung verstanden, die in dem festen, halbfesten, cremigen oder flüssigen Kosmetikprodukt fein verteilt, dispergiert beziehungsweise suspendiert vorliegt. Solche Puderkomponenten haben in der Regel eine Teilchengröße von etwa 0,5 bis 200 µm, insbesondere von 1 bis 100 µm. Sie stellen in der Regel einen Anteil von etwa 0,5 bis 20 Gew.-% des fertigen Kosmetikprodukts dar. Der Puderstoff kann selbst ungefärbt sein oder eine Eigenfarbe aufweisen, beispielsweise Metalloxide als Puderkomponente wie Titandioxid oder Aluminiumoxid. Puderkomponenten vermitteln im aufgetragenen Kosmetikprodukt in der Regel ein mattes, mattglänzendes oder mattierendes Erscheinungsbild. Puderkomponenten dienen, alternativ oder in Ergänzung, dazu je nach ihrer Eigenfärbung und spezifischer Eigenschaft die Farbgebung der in der Zusammensetzung enthaltenen farbgebenden Komponenten wie Farbpigmente zu verstärken oder abzuschwächen. Diese Effekte sind im Wesentlichen auf die Eigenschaft der Puderkomponenten, das Licht im aufgetragenen Produkt diffus zu streuen, zurückzuführen. Alternativ oder in Ergänzung dazu tragen Puderkomponenten in kosmetischen Zusammensetzungen zu einer Verbesserung des Hautgefühls beim Auftragen des kosmetischen Produkts und zur Hauthaftung des Produkts bei. Sie sind, alternativ oder in Ergänzung dazu, als Konsistenz gebendes Füll- und/oder Bindematerial in der kosmetischen Zusammensetzung enthalten.

Die Erfinder fanden überraschend, dass Reisstärke in Kosmetikzusammensetzungen der dekorativen Kosmetik eingesetzt werden kann, um solche Puderkomponenten, die herkömmlicher Weise verwendet werden, wie Wismutchlorid, Mica (auch titanisiert), Aluminium-Stärke-Octenylsuccinat, Siliziumoxid, Polymethylmethacrylat, Teflon, Bornitrid, Acryl-Copolymere, Aluminiumsilikat, Bentonid, Kalziumsilikat, Kalziumkarbonat, Kaolin, Magnesiumaluminiumsilikat, Magnesiumtrisilikat, Talkum, Titandioxid, Zinksalze, Zinkoxid, Aluminiumoxid, Kalziumkarbonat sowie weitere Puder synthetischer Polymere wie PMMA, Nylon-12 und ähnliche Substanzen vollständig zu ersetzen.

Von den Puderkomponenten, die meist als feine Partikel vorliegen, sind die Farbpigmente zu unterscheiden. Sie sind partikelförmige Farbmittel, im Gegensatz zu den meist löslichen Farbstoffen. Sie sind erfindungsgemäß bevorzugt als farbgebende Komponenten zusammen mit der Reisstärke in der kosmetischen Zusammensetzung enthalten. Die Gruppe der anorganischen Farbpigmente wird in erster Linie aus den färbenden Metalloxiden gebildet. Die Gruppe der organischen Farbpigmente enthält in der Regel verschiedene aromatische Verbindungen wie Azofarbstoffe, Indigo und indigoide Farbstoffe, Triphenylmetan, Anthrachinon sowie Xanthinfarbstoffe.

Die Erfinder fanden überraschend, dass Reisstärke in Kosmetikzusammensetzungen der dekorativen Kosmetik eingesetzt werden kann, um eine Steigerung der Deckkraft beziehungsweise der Farbdichte des aufgetragenen kosmetischen Produkts zu erzielen. Besonders vorteilhaft kann so ein besonderer optischer Effekt erzielt werden, der bei herkömmlichen Produkten nur durch mehrfaches Auftragen erreicht wird. Bei einigen Produkten der dekorativen Kosmetik wie Nagellack oder Lippenstift sowie Präparaten zur Überdeckung von Pigmentierungsstörungen der Haut wie Hyperpigmentierung, Erythrem oder Depigmentierung wird in der Regel eine besonders hohe Deckkraft erwünscht, wohingegen bei anderen Produkten wie Lidschatten, Make-up und ähnliche die Natürlichkeit der Anmutung des Farbauftrags durch eine besonders hohe Deckkraft eher vermindert wird. Hier kann aber ein ansprechender optischer Effekt beispielsweise durch entsprechende Verminderung des Anteils der farbgebenden Pigmente im Produkt gegenüber bekannten kosmetischen Zusammensetzungen erzielt werden, wobei gleichzeitig der Anteil potentiell gesundheitsschädlicher Komponenten im Produkt verkleinert wird. Weiter wird erreicht, dass der auslobbare Anteil an natürlichen, insbesondere chemisch unveränderten, nachwachsenden Rohstoffen im Produkt erhöht wird. Darüber hinaus eignet sich die erfindungsgemäß verwendete Reisstärke als physikalischer UV-Filter im Produkt; Reisstärke besitzt ein Absorptionsmaximum bei etwa 290 nm und ist sehr wirksam im UV-B-Bereich (etwa 290 nm bis etwa 320 nm).

Während der Zusatz von beispielsweise Kartoffelstärke oder anderer Getreidestärken wie Maisstärke zu einer puderkomponentenfreien Lippenstiftzusammensetzung den Farbauftrag auf die Haut beziehungsweise die Deckkraft gegenüber der puderkomponentenfreien Zusammensetzung nicht verbessert, führt der Zusatz von Reisstärke, in einem Anteil von 1 bis 20 Gew.-%, bevorzugt von 2 bis 8 Gew.-%, insbesondere bevorzugt von 5 Gew.-%, zu einer deutlichen Verbesserung des Farbauftrags unter normierten Applikationsbedingungen und zu einer Steigerung der Deckkraft des aufgetragenen Produkts gegenüber der puderkomponentenfreien sowie der mit Kartoffelstärke beziehungsweise Maisstärke versetzten Lippenstiftzusammensetzung.

Eine chemische Modifikation der Reisstärke, beispielsweise durch Quervernetzen, zur Beeinflussung der Quelltemperatur, wie beispielsweise bei Kartoffel- beziehungsweise Maisstärke notwendig, kann vorteilhafter Weise bei der erfindungsgemäßen Verwendung von Reisstärke in kosmetischen Zusammensetzungen nicht durchgeführt zu werden. Reisstärke wird daher erfindungsgemäß in nativer, chemisch unmodifizierter Form in kosmetischen Zusammensetzungen eingesetzt. Die Verbesserung des Farbauftrags bei der Anwendung des erfindungsgemäß erhaltenen kosmetischen Zusammensetzung und der Deckkraft ist im Wesentlichen auf einen gleichmäßigeren und dichteren Auftrag des Produkts zurückzuführen, was gleichzeitig und außerdem eine bessere Haftung der aufgetragenen kosmetischen Zusammensetzung bedingt. Ohne an die Theorie gebunden zu sein, bedingt die mikrokristalline Struktur der Reisstärke, welche eckige und kantige kleine Partikel aufweist, eine verbesserte Haftung der Reisstärkepartikel unteren ander sowie eine verbesserte Haftung der kosmetischen Zusammensetzung auf der Oberfläche von Haut, Haaren oder Nägeln und/oder beim Vorgang des Auftragens ein größerer Anteil von der kosmetischen Zusammensetzung Produkt in einer gleichmäßigeren und dickeren Schicht aufgetragen wird. Dieser Effekt macht sich besonders bemerkbar bei in Stiftform ausgebildeten kosmetischen Produkten wie fetthaltige gegossene Produkte und so genannte fettarme oder fettfreie Schminkkreiden. Bevorzugte Produkte dieser Art sind Lippenstifte, Lip-liner, Eye-liner, Kajal- oder Augenbrauen-Stifte. Hier kann schon mit einem einzigen Auftragungsstrich ein gleichmäßig hoher Produktauftrag mit hoher Deckkraft erzielt werden. Die durch die erfindungsgemäße Verwendung von Reisstärke enthaltene kosmetische Zusammensetzung wird bevorzugt eingesetzt als Produkt der dekorativen Kosmetik zur Abschwächung der Erscheinung kosmetischer Fehler der Haut wie Fältchen, Poren, Farbveränderungen, Pigmentstörungen.

Weiter ist die Verwendung in lackartigen, aushärtenden kosmetischen Zusammensetzungen wie Nagelüberzugsmittel beziehungsweise Nagellack vorgesehen. Die Erfinder fanden überraschend, dass auch der Farbauftrag, insbesondere die Gleichmäßigkeit des Auftrags, die Kontrollierbarkeit des Auftrags bei der Anwendung und/oder die Deckkraft von Nagelüberzugsmitteln gegenüber bekannten Nagelüberzugsmitteln verbessert werden kann.

Die erfindungsgemäße Verwendung von Reisstärke zur Verbesserung des Farbauftrags erlaubt dabei die Bereitstellung einer kosmetischen Zusammensetzung, welche bevorzugt im Wesentlichen frei von Metalloxiden ist. Sie erlaubt auch die Bereitstellung einer kosmetischen Zusammensetzung, die bevorzugt im Wesentlichen frei von weiteren Hilfsstoffen, Puderkomponenten und/oder heterozyklischen Verbindungen ist.

Gegenstand der vorliegenden Erfindung ist mithin auch eine kosmetische Gegenstand der vorliegenden Erfindung ist mithin auch eine kosmetische Zusammensetzung, die insbesondere auf Haut, Haare oder Nägel aufgetragen wird und bei der Anwendung einen verbesserten Farbauftrag und insbesondere eine gesteigerte Deckkraft aufweist. Erfindungsgemäß enthält die kosmetische Zusammensetzung mindestens eine farbgebende Komponente, insbesondere Farbpigment, und einen effektiven Anteil an Reisstärke von 1 bis 10 Gew.-%, bevorzugt von 2 bis 8 Gew.-%, insbesondere etwa 5 Gew.-%. Erfindungsgemäß ist in der kosmetische Zusammensetzung der vorliegenden Erfindung Reisstärke als einzige Puderkomponente enthalten. Die Reisstärke eiegt in nativer, chemisch unmodifizierter Form vor.

Bevorzugt ist der Gegenstand der vorliegenden Erfindung ist ausgewählt aus der Gruppe der Produkte der dekorativen Kosmetik, bestehend aus Nagelüberzugsmitteln wie Nagellack, Zusammensetzungen zum Auftragen auf die Haut wie fetthaltige gegossene oder verpresste Produkte sowie fettarme oder fettfreie Schminkkreiden, insbesondere Lippenstift, Make-up, Lip-liner, Eye-liner, Kajal und Lidschatten sowie Zusammensetzungen zum Auftragen auf Gesichtshaare wie Mascara und Augenbrauenstift. In einer bevorzugten Variante ist die kosmetische Zusammensetzung im Wesentlichen frei von Metalloxiden. In einer weiteren Variante ist die kosmetische Zusammensetzung im Wesentlichen frei von heterocyclischen Verbindungen.

In einer bevorzugten Ausführungsform ist die kosmetische Zusammensetzung der dekorativen Kosmetik ein Lippenstift, mit verbessertem Farbauftrag und insbesondere gesteigerter Deckkraft, der die folgenden Komponenten enthält: a) mindestens eine farbgebende Komponente, insbesondere Farbpigment, b) Wachsphase, c) Öl-/Fettphase und d) einen effektiven Anteil an Reisstärke; von 1 Gew.-% bis 20 Gew.-%, wobei die Reisstärke als einzige Puderkomponente in nativer, chemisch ununmodifizierter Form vorliegt.

In einer weiteren bevorzugten Ausführungsform ist die kosmetische Zusammensetzung der dekorativen Kosmetik ein Nagellack, der zumindest folgende enthält: a) mindestens eine farbgebende Komponente, b) mindestens eine Lack bildende Komponente und einen effektiven Anteil an Reisstärke; von 1 Gew.-% bis 10 Gew.%, wobei die Reisstärke als einzige Puderkomponente in nativer, chemisch unmodifizierter Form vorliegt.

Es ist erfindungsgemäß aber auch vorgesehen, dass die beanspruchte kosmetische Zusammensetzung noch weitere für entsprechende Produkte übliche Bestandteile und Hilfsstoffe ausweist. Dazu zählen beispielsweise Stoffe zur weiteren Verbesserung der Haftung des Produkts auf der Haut-, Haarbeziehungsweise Nageloberfläche wie Metallstearate, beispielsweise Zink-, Kalzium-, Magnesium- oder Aluminiumstearat, oder Stoffe, die das aufgetragene Produkt seidig oder perlmuttschillernd erscheinen lassen, wie Wismutoxychlorid, Titandioxid, Glimmer oder Aluminiumpulver.

Ein weiterer Gegenstand der vorliegenden Erfindung ist schließlich ein Verfahren zur Herstellung einer kosmetischen Zusammensetzung, die einen verbesserten Farbauftrag aufweist und insbesondere auf Haut, Haare oder Nägel aufgetragen wird. In einem in dem erfindungsgemäßen Verfahren enthaltenen Verfahrensschritt wird einer kosmetischen Zusammensetzung, die frei von Puderkomponenten ist, ein effektiver Anteil an Reisstärke zugefügt und daraus eine kosmetische Zusammensetzung mit verbesserten Eigenschaften in Bezug auf den Farbauftrag erhalten wird. Der erfindungsgemäß zugefügte effektive Anteil an nativer, chemisch unmodifizierter Reisstärke beträgt von 1 bis 10 Gew.-%, bevorzugt von 2 bis 8 Gew.-%, besonders bevorzugt etwa 5 Gew.-%.

Die nachstehenden Figuren und Beispiele sollen die Erfindung näher erläutern, ohne sie jedoch einzuschränken.

Die Figuren zeigen:

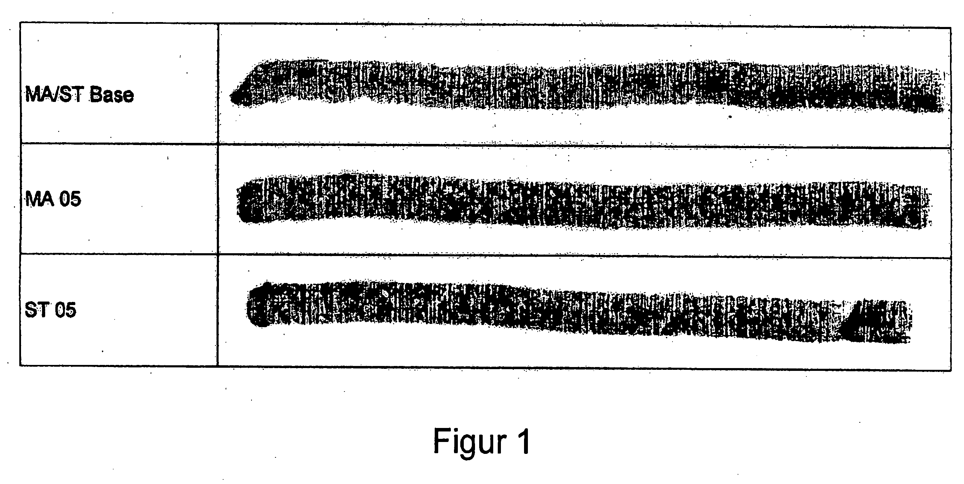

Figur 1 Ergebnis des normierten Auftragungsversuchs mit einer stiftförmigen Kosmetikzusammensetzung (Lippenstift): obere Spur: puderkomponentenfreie farbmittelhaltige Zusammensetzung (Grundstoff), mittlere Spur: farbmittelhaltige Zusammensetzung mit Zusatz von 5 Gew.-% Maisstärke (MA 05), untere Spur: farbmittelhaltige Zusammensetzung mit Zusatz von 5 Gew.-% Kartoffelstärke (ST 05).

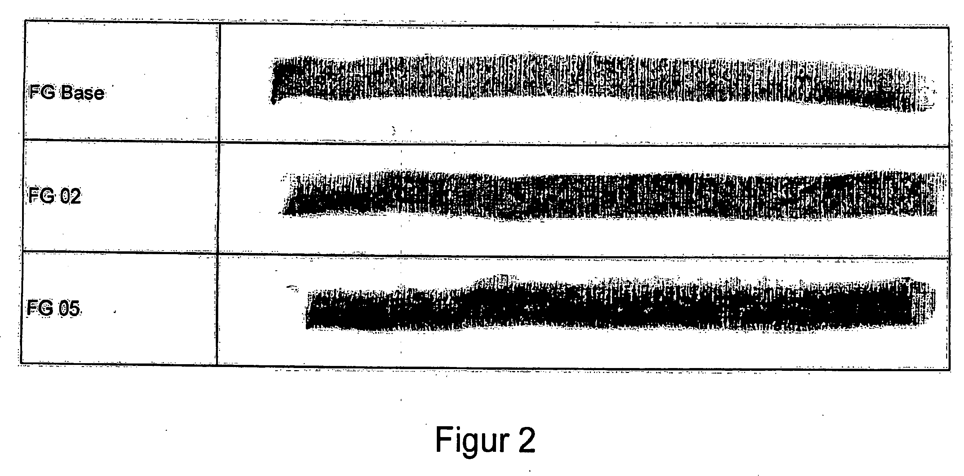

Figur 2 Ergebnis des normierten Auftragungsversuchs mit einer stiftförmigen Kosmetikzusammensetzung (Lippenstift): obere Spur: puderkomponentenfreie farbmittelhaltige Zusammensetzung (Grundstoff), mittlere Spur: erfindungsgemäße farbmittelhaltige Zusammensetzung mit Zusatz von 2 Gew.-% Reisstärke (FG 02), untere Spur: erfindungsgemäße farbmittelhaltige Zusammensetzung mit Zusatz von 5 Gew.-% Reisstärke (FG 05).

### Beispiel 1:

### Herstellung einer stiftförmigen Kosmetikzusammensetzung mit Reisstärke

### 1.1 Bereitstellung der Reisstärke

Als Ausgangsmaterial wird gereinigter, polierter und gebrochener Reis verwendet, woraus die Stärke extrahiert wird. Nach der Extraktion wird die Stärke aufgereinigt. Die so erhaltene Stärke liegt in fein vermahlener Form vor. Ein Produkt dieser Art ist beispielsweise die Reisstärke Typ FG-KA der Firma Remy Industries n.v., Belgien. Diese besitzt eine Partikelgröße zwischen 2 und 8 µm in Suspension. Weiter weisen die Stärkekristalle der Reisstärke eine "raue" oder "kantige" Oberfläche auf. Im Vergleich dazu besitzen beispielsweise Maisstärke eine Partikelgröße zwischen etwa 20 und 30 µm und Kartoffelstärke eine Partikelgröße zwischen 80 und 100 µm. Bei Maisstärke und Kartoffelstärke weisen die Stärkepartikel eine vergleichsweise glatte Oberfläche auf. Die gewählte unmodifizierte, native Reisstärke besitzt eine Verkleisterungstemperatur im Bereich von etwa 60°C bis 80°C. Die Quelltemperatur beziehungsweise Verkleisterungstemperatur kann durch Wahl der Qualität beziehungsweise der Sorte der Reisstärke variiert werden. Besonders vorteilhaft liegt die Reisstärke bei Temperaturen von etwa 37°C unverkleistert und damit in feiner Verteilung oder Suspension vor.

Gemäß Produktanalyse besitzt die gewählte Reisstärke Nennwerte von maximal 14 % Restfeuchtigkeit, maximal 0,6 Gew.-% (Trockensubstanz) Asche, maximal 0,55 Gew.-% (Trockensubstanz) Protein, maximal 0,1 Gew.-% (Trockensubstanz) Fett und mindestens 97 Gew.-% (Trockensubstanz) Stärke. Der Metallgehalt beträgt in der Regel weniger als 2500 ppm Natrium, weniger als 10 ppm Eisen und weniger als 100 ppm Kalzium. Der pH-Wert liegt im Bereich von 6 bis 7,5 bei einer Azidität von maximal 2,0 ml 0,mol/l NaOH pro 5 g.

### 1.2 Kosmetikprodukt

Es werden fünf verschiedene Kosmetikprodukte gemäß den Zusammensetzungen in Tabelle 1 hergestellt und in Stiftform gegossen.

Die Grundmasse für stiftförmigen Kosmetikprodukts besteht aus einer Öl-/Fettphase, die etwa 70 % des Gesamtprodukts ausmacht, und aus einer Wachsphase. Beide Phasen weisen einen Schmelzpunkt von etwa 60 bis 75°C auf.

**Tabelle 1:**

| **Handels-name** | **INCI-Name** | **FG/MA/ ST Base** | **FG 02(*** | **FG 05(*** | **MA 05** | **ST 05** |
|---|---|---|---|---|---|---|
| Wachsphase: | | | | | | |
| Carnauba Wax 2442 L | Cera Carnauba | 2,55 | 2,50 | 2,42 | 2,42 | 2,42 |
| Candelilla Wax 2039 L | Candelilla Cera | 7,55 | 7,40 | 7,17 | 7,17 | 7,17 |
| TeCe Ozokerit HH weiß | Ozokerite | 370 | 3,63 | 3,52 | 3,52 | 3,52 |
| Tegosoft SH | Stearyl Heptanoate | 0,50 | 0,49 | 0,47 | 0,47 | 0,47 |
| Abil Wax 2434 | Stearoxy Dimethicone | 0,75 | 0,74 | 0,71 | 0,71 | 0,71 |
| Abil Wax 2440 | Behenoxy Dimethicone | 0,75 | 0,74 | 0,71 | 0,71 | 0,71 |
| Rewopal PIB 1000 | Polyisobutene | 5,00 | 4,90 | 4,75 | 4,75 | 4,75 |
| Antaron V 220 | PVP/Eicosen e Copolymer | 0,25 | 0,25 | 0,25 | 0,25 | 0,25 |

| Fett-/Öl-Phase: | | | | | | |
|---|---|---|---|---|---|---|
| Adeps Lanae Lite | Lanolin | 32,70 | 32,02 | 31,07 | 31,07 | 31,07 |
| Crodamol ML / Ceraphyl 50 | Myristyl Lactate | 8,25 | 8,09 | 7,84 | 7,84 | 7,84 |
| Rizinusöl DAB 10 | Ricinus Communis | 6,70 | 6,19 | 5,42 | 5,42 | 5,42 |
| Tegosoft Liquid / Luvitol EHO | Cetearyl Ethylhexanoate | 12,48 | 12,23 | 11,85 | 11,85 | 11,85 |
| Oxynex K liquid | Asorbyl Palmitate, Tocopherol, Ascorbic Acid, Citric Acid, PEG-8 | 0,02 | 0,02 | 0,02 | 0,02 | 0,02 |

| Puderkomponente: | | | | | | |
|---|---|---|---|---|---|---|
| Remy FG-KA | Oryza sativa | - | 2,00 | 5,00 | - | - |
| Maisstärke | Zea mays | - | - | - | 5,00 | - |
| Kartoffelstärke | Solanum tuberosum | - | - | - | - | 5,00 |

| Farbpigment: | | | | | | |
|---|---|---|---|---|---|---|
| Covapate Blanc W 9765 | CI 77891, Castor Oil | 2,50 | 2,50 | 2,50 | 2,50 | 2,50 |
| Covapate Rubis W 4765 | CI 15850, Castor Oil | 9,30 | 9,30 | 9,30 | 9,30 | 9,30 |
| Covapate Orange W 2762 | CI 15985, Castor Oil | 7,00 | 7,00 | 7,00 | 7,00 | 7,00 |
| Gesamt [g]: | | 100,00 | 100,00 | 100,00 | 100,00 | 100,00 |

| | | | | | | |
|---|---|---|---|---|---|---|
| *) erfindungsgemäß | | | | | | |

### Beispiel 2:

### Eigenschaften der erfindungsgemäßen Kosmetikzusammensetzung

Die in Beispiel 1 hergestellten Kosmetikprodukte werden auf ihre Eigenschaften bei der kosmetischen Anwendung untersucht. Dazu werden die stiftförmigen Produkte in einem normierten Auftragungsversuch unter einem bestimmten Druck auf die Haut oder eine Oberfläche mit gleicher Rauheit mit einer bestimmten Ziehgeschwindigkeit aufgebracht.

Danach wird die auf dieser Oberfläche aufgebrachte Menge an Kosmetikprodukt, die Gleichmäßigkeit des Farbauftrags sowie die Deckkraft des aufgetragenen Produkts analysiert.

In den Figuren 1 und 2 sind die gefundenen Ergebnisse dargestellt. So zeigt ein nach dem Stand der Technik zusammengesetztes Kosmetikprodukt, das keine Puderkomponente aufweist ("Base FG/MA/ST"), bei den gewählten Einstellungen einen mittleren Farbauftrag (obere Spur von Figur 1). Der Zusatz von 5 Gew.-% Maisstärke (Figur 1, mittlere Spur) beziehungsweise 5 Gew.-% Kartoffelstärke (Figur 1, untere Spur) als Puderkomponente im Kosmetikprodukt führt bei gleichen Bedingungen des Auftrags zu keinerlei messbarer Verbesserung des Farbauftrags, jeweils gemessen als Menge und Deckkraft des aufgetragenen Produkts. Demgegenüber zeigen Kosmetikprodukte mit der erfindungsgemäßen Zusammensetzung ("FG 02, FG 05") bei gleichen Bedingungen des Auftrags eine gegenüber dem puderkomponentenfreien Kosmetikprodukt (Figur 2, obere Spur) einen deutlich verbesserten Farbauftrag und eine verbesserte Deckkraft des aufgetragenen Produkts (Figur 2, mittlere und untere Spur), jeweils gemessen als Menge und Deckkraft des aufgetragenen Produkts.

## Patentansprüche

1. Verwendung von Reisstärke zur Verbesserung des Farbauftrags bei kosmetischen Zusammensetzungen der dekorativen Kosmetik.

2. Kosmetische Zusammensetzung der dekorativen Kosmetik mit verbessertem Farbauftrag, enthaltend:
a) mindestens eine farbgebende Komponente und
b) einen effektiven Anteil an Reisstärke von 1 Gew.-% bis 10 Gew.-%,
wobei die Reisstärke als einzige Puderkomponente in der Zusammensetzung enthalten ist und in nativer, chemisch unmodifizierter Form vorliegt.

3. Kosmetische Zusammensetzung nach Anspruch 2, wobei der effektive Anteil an Reisstärke von 2 Gew.-% bis 8 Gew.-% beträgt.

4. Kosmetische Zusammensetzung nach einem der Ansprüche 2 oder 3, ausgewählt aus Nagelüberzugsmitteln wie Nagellack, Zusammensetzungen zum Auftragen auf die Haut wie Lippenstift, Make-up, Lip-liner, Eye-liner, Kajal und Lidschatten und Zusammensetzungen zum Auftragen auf Gesichtshaare wie Mascara und Augenbrauenstift.

5. Lippenstift enthaltend:
a) mindestens eine farbgebende Komponente,
b) eine Wachsphase,
c) eine Öl-/Fettphase und
d) einen effektiven Anteil an Reisstärke von 1 Gew.-% bis 20 Gew.-%, wobei die Reisstärke als einzige Puderkomponente in nativer, chemisch ununmodifizierter Form vorliegt.

6. Nagellack enthaltend:
a) mindestens eine farbgebende Komponente,
b) mindestens eine Lack bildende Komponente und
c) einen effektiven Anteil an Reisstärke von 1 Gew.-% bis 10 Gew.%, wobei die Reisstärke als einzige Puderkomponente in nativer, chemisch unmodifizierter Form vorliegt.

7. Verfahren zur Herstellung einer Zusammensetzung der dekorativen Kosmetik mit verbessertem Farbauftrag, wobei einer kosmetischen Zusammensetzung ein effektiver Anteil an nativer, chemisch unmodifizierter Reisstärke von 1 Gew.-% bis 10 Gew. % als einzige Puderkomponente der Zusammensetzung zugefügt wird und eine kosmetische Zusammensetzung mit verbessertem Farbauftrag und gesteigerter Deckkraft erhalten wird.

## Claims

1. A use of rice starch for improving the color application of cosmetic compositions of decorative cosmetics.

2. A cosmetic composition of decorative cosmetics with improved color application, containing:
a) at least one color providing component, and
b) an effective percentage of rice starch of 1 percent by weight to 10 percent by weight,
wherein the rice starch is the only powder component contained in the composition and is present in its native, chemically unmodified form.

3. The cosmetic composition according to Claim 2, wherein the effective percentage of rice starch is 2 percent by weight to 8 percent by weight.

4. The cosmetic composition according to any one of Claims 2 or 3, selected from nail coating means such as nail polish, compositions for application to the skin such as lipstick, make-up, lip liner, eye liner, kohl and eye shadow, and compositions for application on hair in the face such as mascara and eyebrow pencil.

5. A lipstick, containing
a) at least one color providing component,
b) a waxy phase,
c) an oily/fatty phase, and
d) an effective percentage of rice starch of 1 percent by weight to 20 percent by weight, wherein the rice starch is the only powder component and is present in its native, chemically unmodified form.

6. A nail polish, containing:
a) at least one color providing component,
b) at least one varnish forming component, and
c) an effective percentage of rice starch of 1 percent by weight to 10 percent by weight, wherein the rice starch is the only powder component and is present in its native, chemically unmodified form.

7. A method for the manufacture of a composition of decorative cosmetics with improved color application, wherein an effective percentage of native, chemically unmodified rice starch of 1 percent by weight to 10 percent by weight is added to a cosmetic composition as the only powder component of the composition and a cosmetic composition with improved color application and increased covering power is obtained.

## Revendications

1. Utilisation d'amidon de riz pour l'amélioration de l'application de couleur dans le cas de compositions cosmétiques de la cosmétique décorative.

2. Composition cosmétique de la cosmétique décorative avec application de couleur améliorée, contenant :
a) au moins un composant chromophore et
b) une proportion efficace d'amidon de riz de 1 % en poids à 10 % en poids,
dans laquelle l'amidon de riz est contenu dans la composition en tant qu'unique composant de poudre et est présent sous forme native, non modifiée chimiquement.

3. Composition cosmétique selon la revendication 2, dans laquelle la proportion efficace d'amidon de riz va de 2 % en poids à 8 % en poids.

4. Composition cosmétique selon l'une quelconque des revendications 2 ou 3, sélectionnée parmi des agents de recouvrement d'ongles comme le vernis à ongles, des compositions pour l'application sur la peau comme le rouge à lèvres, le maquillage, le crayon à lèvres, l'eye-liner, le khôl et des ombres à paupières et des compositions pour l'application sur les poils faciaux comme le mascara et le crayon à sourcils.

5. Rouge à lèvres contenant :
a) au moins un composant chromophore,
b) une phase de cire,
c) une phase huileuse/grasse et
d) une proportion efficace d'amidon de riz de 1 % en poids à 20 % en poids, dans lequel l'amidon de riz est présent en tant qu'unique composant de poudre sous forme native, non modifiée chimiquement.

6. Vernis à ongles contenant :
a) au moins un composant chromophore,
b) au moins un composant de formation de vernis et
c) une proportion efficace d'amidon de riz de 1 % en poids à 10 % en poids, dans lequel l'amidon de riz est présent en tant qu'unique composant de poudre sous forme native, non modifiée chimiquement.

7. Procédé de fabrication d'une composition de la cosmétique décorative avec application de couleur améliorée, dans lequel une proportion efficace d'amidon de riz natif, non modifié chimiquement de 1 % en poids à 10 % en poids est ajoutée à une composition cosmétique en tant qu'unique composant de poudre de la composition et une composition cosmétique avec application de couleur améliorée et pouvoir couvrant accru est obtenue.
